# EUROPEAN PATENT APPLICATION

(11) **EP 2 617 436 A1**
(43) Date of publication of application: **24.07.2013**
(21) Application number: 13380001.1
(22) Date of filing: 17.01.2013
(51) Int. Cl.: A61L 9/14, A61L 9/12

(54) **Air freshening device for enclosures**

(30) Priority: 19.01.2012 ES 201230055 U
(71) Applicant: Salcedo Bonilla, Julio, 08005 Barcelona (ES)
(72) Inventor: Salcedo Bonilla, Julio, 08005 Barcelona (ES)
(74) Representative: Espiell Volart, Eduardo Maria

(57) **Abstract**

It comprises a set of impeller pumps -1-, the suction conduits -2- of which are connected to the corresponding fragrance-containing receptacles -4-, the impeller outlets -3- of which are connected to connections -5- of irrigation pipes -7- that extend toward the different atomising nozzles -8- disposed in high places, preferably ceilings -9-, of the enclosures, all of which is disposed in the interior of a protective casing -6-, having on its lateral side displays -10- for observing the level of the liquid in the containers -4- and luminous indicators that indicate the operation of the corresponding pump -1-. It also has activation and control elements consisting of a digital programmer -12-, a timer -13- and a manual dashboard -14-, fixed or remotely controlled.

## Description

The object of the present invention is a device for freshening large spaces or enclosures, which is carried out by nebulising and atomising predetermined fragrances, thereby achieving a personalised aromatisation of said spaces permanently or temporarily. The essential characteristics of the aforementioned device are described below.

### OBJECT OF THE INVENTION

The device being described is intended for use in large enclosures, such as auditoria, shopping centres, certain specialised stores such as clothes boutiques and perfumeries, as well as fashion show venues.

In many cases, different areas of these enclosures can be freshened in accordance with the products displayed for sale, as in the case of clothes and similar stores, offering different fragrances in the men's and women's section, for example.

In the case of auditoria, theatres or cinemas, the development of a computer program for activating the device at certain moments of the performance or film to offer fragrances in accordance with the scene (for example, a fire, an explosion or a country landscape), with matching aromas and similar effects for other places of application, including the promotion of the fragrance itself by installing the device in a structure whereunder the public attending the enclosure must pass and will come into contact with the fragrance being promoted.

### CURRENT STATE OF THE ART

At present, the existence of similar devices which can offer the applications described in the previous paragraphs in an automated and previously programmed manner is unknown, due to which it is considered that the essential characteristics of the device object of the present invention cover a broad spectrum of applications.

### DESCRIPTION OF THE DEVICE OBJECT OF THE INVENTION

The air freshening device for large enclosures object of the present invention essentially consists of a set of impeller pumps of different sizes and capacities in accordance with their use and place of application, whether for use in large enclosures or of smaller size, as required.

These pumps are directly in contact with the corresponding air freshener liquid receptacles, which have different aromatic fragrances and capacities in accordance with their application, wherein the pumps are also connected to the conduits (hereinafter referred to as "irrigation pipes"), which end in the corresponding atomising nozzles, preferably disposed on the ceiling or high places of the enclosure, which will nebulise the aroma in such a manner that, while extremely small, the drops are not perceived, creating the desired effect of a pleasant sensation similar to a breeze.

The set of impeller pumps and fragrance receptacles is adequately disposed in the interior of a protective casing large enough to house all of the aforementioned content. Said casing has the necessary connections for the irrigation pipes that extend therefrom, adequately installed, toward the places where the atomising nozzles are installed, said connections being connected, in turn, to the impeller pump outlets.

The casing is equipped with openings, by way of display, for controlling the level of liquid in each of the fragrance receptacles, and luminous indicators, preferably LEDs, indicating the operation of the corresponding impeller pump.

The device is completed with the activation elements, consisting of a digital programmer which enables, upon introducing the desired program, the emission of the adequate fragrance(s) at the desired times.

This activation can be carried out by means of a remote control or manual dashboard, said activation elements being completed with a 24-hour timer that controls the different activation channels, causing the emission of the fragrance exactly at the desired time.

### GRAPHIC REPRESENTATION

As a complement to the foregoing description and by way of example, a drawing is included wherein:

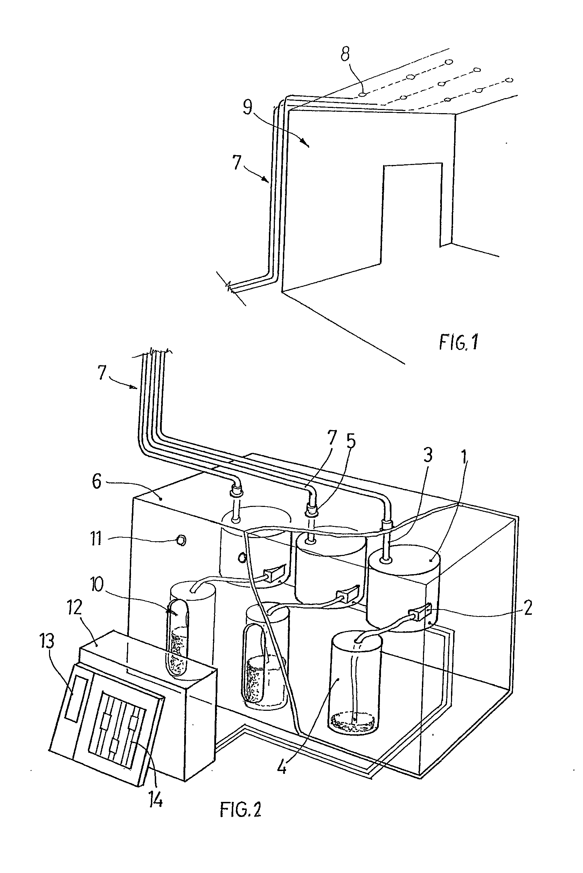

- fig. 1: shows a schematic perspective view of an installation of the conduits disposed for freshening the air of an enclosure; and
- fig. 2: shows the air freshening device object of the present invention, also in a perspective view.

According to this drawing, the air freshening device for large enclosures essentially consists of a set of impeller pumps -1-, of the suction-impelling type, wherein their suction conduits -2- are connected to receptacles -4- containing different fragrances, while the aforementioned pumps -1- are connected at their impelling outlets -3- to connections -5- in the exterior of the casing -6-, whereto irrigation conduits -7- are fixed that extend toward different atomising nozzles -8-, preferably installed in high places such as the ceilings -9- of the enclosures to be aromatised.

The casing -6-, wherein the impeller pumps -1- and fragrance receptacles -4- are housed, together with the different connections and conduits -7-, have displays -10- on the external face thereof for observing and controlling the content of the receptacles -4-, as well as luminous LEDs -11- that indicate the operation of the respective impeller pumps -1-.

A digital programmer -12-, together with a timer -13- and a manual dashboard -14-, fixed or remotely controlled, constitute the activation assembly of the described device.

It should be noted, by way of conclusion of the foregoing, that any variation in the dimensions, sizes, appearance and finishes, as well as in the types of materials used in the practical embodiment of the device, will not alter the essentiality of the invention, which is summarised in the following claims

## Claims

1. Air freshening device for enclosures, **characterised in that** it comprises a set of impeller pumps -1- for impelling liquids, having adequate dimensions and capacities, the suction conduits -2- of which are connected to the corresponding receptacles -4- that contain liquid aromas or fragrances, of varying sizes in accordance with the application thereof, the impeller outlets -3- of which are connected to connections -5- of irrigation pipes -7- that extend toward different atomising nozzles -8- disposed in high places, preferably in the ceilings -9- of the enclosures to be freshened.

2. Air freshening device for enclosures, according to the preceding claim, **characterised in that** the set of impeller pumps -1- and fragrance-containing receptacles -4-, together with the different connections -5- and pipes -7-, is disposed in the interior of a sufficiently large protective casing -6- having on its lateral side displays -10- for viewing the level of the liquid in the containers -4- and luminous indicators, preferably LEDs -11- indicating the operation of the corresponding pump -1-.

3. Air freshening device for enclosures, according to claims 1 and 2, **characterised in that** it has activation and control elements consisting of a digital programmer -12- for selecting the desired sequence programs, times and types of fragrances, according to the place and application, together with a timer -13- and a manual dashboard -14-, fixed or remotely controlled.
